(19) Europäisches Patentamt
European Patent Office
Office européen des brevets

(11) **EP 4 443 156 A1**

(12) **EUROPEAN PATENT APPLICATION**
published in accordance with Art. 153(4) EPC

(43) Date of publication:
**09.10.2024 Bulletin 2024/41**

(21) Application number: **22924958.6**

(22) Date of filing: **21.11.2022**

(51) International Patent Classification (IPC):
*G01N 30/08* (2006.01)    *C10B 57/04* (2006.01)
*G01N 27/62* (2021.01)    *G01N 30/06* (2006.01)
*G01N 30/72* (2006.01)    *G01N 33/22* (2006.01)

(52) Cooperative Patent Classification (CPC):
**C10B 57/04; G01N 27/62; G01N 30/06;
G01N 30/08; G01N 30/72; G01N 33/22**

(86) International application number:
**PCT/JP2022/042941**

(87) International publication number:
**WO 2023/149053 (10.08.2023 Gazette 2023/32)**

(84) Designated Contracting States:
**AL AT BE BG CH CY CZ DE DK EE ES FI FR GB
GR HR HU IE IS IT LI LT LU LV MC ME MK MT NL
NO PL PT RO RS SE SI SK SM TR**
Designated Extension States:
**BA**
Designated Validation States:
**KH MA MD TN**

(30) Priority: **07.02.2022 JP 2022016874**

(71) Applicant: **JFE Steel Corporation
Tokyo 100-0011 (JP)**

(72) Inventors:
• **TANDOKORO Kohei**
  **Tokyo 100-0011 (JP)**
• **TAMURA Shunsuke**
  **Tokyo 100-0011 (JP)**
• **ISHIDA Tomoharu**
  **Tokyo 100-0011 (JP)**

(74) Representative: **Grünecker Patent- und
Rechtsanwälte
PartG mbB
Leopoldstraße 4
80802 München (DE)**

(54) **METHOD FOR QUANTIFYING GAS GENERATED FROM COAL, METHOD FOR ESTIMATING AMOUNT OF GAS GENERATED DURING COAL CARBONIZATION, AND METHOD FOR MANUFACTURING COKE**

(57)     Gas generated during heating of coal is collected. The collected gas is introduced into a separation column 3 of a gas chromatograph. The gas introduced into the separation column 3 is captured by cooling the separation column 3. The captured gas is released by deactivating the cooling of the separation column 3. At this time, it is preferred to raise a temperature to heat the separation column 3 the cooling of which has been deactivated. The released gas is analyzed and quantified by a mass spectrometry unit 7. With this, gas generated during heating of coal can be quantified with high accuracy.

FIG. 1

**Description**

TECHNICAL FIELD

The present invention relates to a method for quantifying gas generated from coal, a method for estimating an amount of gas generated during coal carbonization, and a method for manufacturing coke.

BACKGROUND ART

Coal is used as an important fossil fuel even in modern times because of the length of the reserve-to-production ratio, the low price, and the like of the coal. Coal is mainly used as a raw material of coke used for ironmaking and as a fuel for thermal power plants, and the amount of consumption is continuously increasing.

One of problems in utilization of coal is handling of gas generated during heating.

In particular, a sulfur-based gas derived from sulfur (S) contained as an impurity of coal is not only highly toxic, but also said to contribute to acid rain and a greenhouse effect. Therefore, the sulfur-based gas is required to be strictly controlled, for example, a regulation value is provided.

In Japan, coke ovens that manufacture coke by carbonization of coal and coal-fired power plants are provided with high-performance desulfurization facilities.

In general, sulfur in coal is regarded as an impurity, and the total sulfur concentration (Total-S, T-S) is one of important indicators of coal quality.

Needless to say, high-quality coal with a low T-S is required for utilization of coal, while coal price has been continuously increasing mainly for coal with a low sulfur concentration (low-sulfur coal) due to recent increase in demand for coal.

In addition, low-sulfur coal is produced only in some limited regions such as Australia, leading to concentration on a specific brand, which may cause a risk that stable procurement becomes difficult due to the influences of weather-induced troubles in a production area, supply and demand trends in foreign countries, and the like.

From such a background, the use of inexpensive coal having a high sulfur concentration (high-sulfur coal) has been under consideration. However, the use of high-sulfur coal means that the amount of sulfur-based gas generated per unit usage amount increases.

In this case, a large load is applied to the existing desulfurization facility, which leads to an increase in desulfurization cost. In a case where a sulfur-based gas equal to or greater than desulfurization capacity of the desulfurization facility is generated, the sulfur-based gas is released to the outside, and a problem of environmental pollution may occur.

Therefore, it is important to figure out a generation temperature (generation temperature range) and a generation amount of sulfur-based gas generated during coal carbonization for each brand of coal, and then select coal as a raw material of coke or a fuel for a thermal power plant.

The generation temperature (generation temperature range) and the generation amount of sulfur-based gas generated during heating of coal depend on the existence form of sulfur in the coal.

Sulfur in coal exists in various forms such as inorganic sulfur and organic sulfur, and the ratio thereof also varies depending on the brand of coal.

Examples of the inorganic sulfur include two kinds, i.e., sulfate sulfur and pyrite sulfur.

Examples of the organic sulfur include thiophene, sulfide, thiol, and sulfonic acid.

Patent Literature 1 discloses a technique of "raising a temperature from room temperature to 1000°C to heat coal in an inert gas, continuously monitoring and measuring sulfur-containing gas generated by thermal decomposition of a sulfur compound, thereby quantifying sulfur in the coal according to its existence form" ([Claim 1]).

More specifically, in the technique disclosed in Patent Literature 1, the gas is monitored using, for example, "mass spectrometry" ([0033]).

CITATION LIST

PATENT LITERATURE

Patent Literature 1: JP 2009-257920 A

SUMMARY OF INVENTION

TECHNICAL PROBLEMS

In the case of quantifying a sulfur-based gas using "mass spectrometry" in the technique disclosed in Patent Literature 1, there is a possibility that evaluation cannot be performed correctly due to superposition with a hydrocarbon-based gas containing no sulfur.

The present invention has been made in view of the above points, and an object of the present invention is to quantify gas generated during heating of coal (particularly, sulfur-based gas) with high accuracy.

SOLUTION TO PROBLEMS

The present inventors have conducted intensive studies, and as a result, have found that employing the configuration described below enables the achievement of the above-mentioned object, thereby completing the present invention.

That is, the present invention provides the following [1] to [6].

[1] A method for quantifying gas generated from coal, comprising:

a gas collection step of collecting gas generated during heating of coal;
a gas introduction step of introducing the gas collected in the gas collection step into a separation column of a gas chromatograph;
a gas capturing step of capturing the gas introduced into the separation column by cooling the separation column;
a gas releasing step of releasing the gas captured in the gas capturing step by deactivating the cooling of the separation column; and
an analysis step of analyzing and quantifying the gas released in the gas releasing step by a mass spectrometry unit.

[2] The method for quantifying gas generated from coal according to [1] above, further comprising a temperature rise heating step of raising a temperature to heat the separation column the cooling of which has been deactivated.

[3] The method for quantifying gas generated from coal according to [1] or [2] above, wherein the gas contains a sulfur-based gas.

[4] A method for estimating an amount of gas generated during coal carbonization, comprising estimating a generation temperature or generation temperature range and a generation amount of a sulfur-based gas generated during coal carbonization based on a quantitative result obtained by the method for quantifying according to [3] above.

[5] A method for manufacturing coke by subjecting coal to carbonization, wherein a manufacturing condition of coke is modified based on an estimation result obtained by the method for estimating according to [4] above.

[6] The method for manufacturing coke according to [5] above, wherein the manufacturing condition is a coal blending ratio.

ADVANTAGEOUS EFFECTS OF INVENTION

According to the present invention, gas generated during heating of coal can be quantified with high accuracy.

BRIEF DESCRIPTION OF DRAWINGS

[FIG. 1] FIG. 1 is a schematic view illustrating an apparatus used for quantification of gas generated from coal.
[FIG. 2] FIG. 2 is a graph showing an amount of hydrogen sulfide ($H_2S$) generated in each heating temperature range.
[FIG. 3] FIG. 3 is a graph showing an amount of sulfur dioxide ($SO_2$) generated in each heating temperature range.

DESCRIPTION OF EMBODIMENTS

[Method for quantifying gas generated from coal]

A method for quantifying gas generated from coal according to the present embodiment (hereinafter, also referred to as "present quantification method" for convenience) includes: a gas collection step of collecting gas generated during heating of coal; a gas introduction step of introducing the gas collected in the gas collection step into a separation column of a gas chromatograph; a gas capturing step of capturing the gas introduced into the separation column by cooling the separation column; a gas releasing step of releasing the gas captured in the gas capturing step by deactivating the cooling of the separation column; and an analysis step of analyzing and quantifying the gas released in the gas releasing step by a mass spectrometry unit.

<Apparatus used for quantification of gas generated from coal>

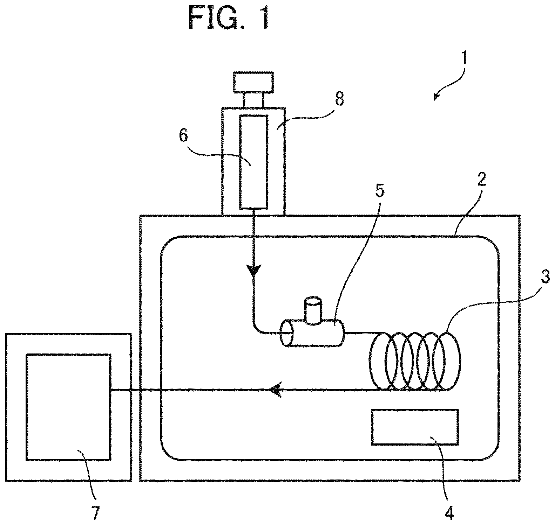

FIG. 1 is a schematic view illustrating an apparatus 1 used for quantification of gas generated from coal.

The apparatus 1 includes a chamber 2. A separation column 3 of a gas chromatograph, a heating unit 4 heating the separation column 3, and a cooling unit 5 cooling at least a part, in a length direction, of the separation column 3 are provided inside the chamber 2.

The apparatus 1 further includes a gas injection unit 6 injecting gas into the separation column 3, and a mass spectrometry unit 7 separating and specifying a gas component contained in the gas introduced from the separation column 3 according to a mass-to-charge ratio.

The heating method of the heating unit 4 is not particularly limited as long as the temperature can be controlled when the separation column 3 is heated, and examples thereof include a heating furnace method using a resistance heater and a high-frequency induction heating method.

The cooling method of the cooling unit 5 is not particularly limited as long as at least a part of the separation column 3 can be cooled and controlled to an arbitrary temperature, and examples thereof include conventionally known methods such as a Peltier cooling method and a liquid nitrogen cooling method.

The cooling range of the cooling unit 5 is not particularly limited, but it is preferable that at least a range of a length of about 10 mm in the separation column 3 can be cooled.

One favorable example of the cooling unit 5 as above is a cryotrap. The cryotrap is a facility that is disposed in the middle of the separation column 3 and blows nitrogen gas cooled with liquid nitrogen to the separation column 3 from the outside.

In the case of using a cryotrap as the cooling unit 5, since the separation capacity by the cryotrap is large, the type and polarity of the separation column 3 may be freely selected.

The apparatus 1 may further include a pyrolysis furnace 8. Gas is generated from coal by heating the coal using the pyrolysis furnace 8.

The pyrolysis furnace 8 may be any furnace as long as it has heating capability corresponding to the heating temperature of the coal to be charged into the pyrolysis furnace 8. As the heating method of the pyrolysis furnace 8, a conventionally known method can be adopted, and examples thereof include a filament type in which heating is performed by a filament, an induction heating type in which a sample foil made of alloy is heated by a high-frequency magnetic field, and a heating furnace type.

Hereinafter, each step included in the present quantification method performed using the apparatus 1 will be described in detail.

<Gas collection step>

The gas collection step is a step of collecting gas generated during heating of coal.

In the gas collection step, it is preferable that coal is charged into the pyrolysis furnace 8 and heated. In this way, gas generated from the coal heated inside the pyrolysis furnace 8 is collected.

The atmosphere in the furnace of the pyrolysis furnace 8 is preferably an inert gas atmosphere in order to avoid superposition of peaks in the mass spectrometry unit 7 in the case of simulating the atmosphere during coal carbonization in a coke oven. The inert gas is preferably helium (He), and may be nitrogen ($N_2$) or the like.

<<Coal>>

Coal to be charged into the pyrolysis furnace 8 is preferably pulverized to a certain grain size or less in order to prevent temperature unevenness during heating. Specifically, the grain size of the coal is preferably 250 um or less and more preferably 125 um or less. When the grain size of the coal is too large, uniform heating is difficult, and unevenness in the amount of gas generated occurs, so that the analysis accuracy in the mass spectrometry unit 7 may be deteriorated.

In order to reduce the influence of moisture in the mass spectrometry unit 7, it is preferable to use well-air-dried coal.

The amount of coal corresponding to the volume of the pyrolysis furnace 8 is charged into the pyrolysis furnace 8. However, when the amount of coal to be charged into the pyrolysis furnace 8 is too large, there is a risk that a large amount of gas is captured in the separation column 3 by use of the cooling unit 5 and the separation column 3 is clogged. Therefore, the amount of coal to be charged into the pyrolysis furnace 8 is preferably about 70 to 80% of the volume of the pyrolysis furnace 8.

<<Gas generated during heating of coal>>

The gas generated during heating of coal contains hydrogen and hydrocarbons ($C_mH_n$) having various molecular weights. Examples of low-molecular-weight hydrocarbons include methane ($CH_4$), ethane ($C_2H_6$), and ethylene ($C_2H_4$). Examples of high-molecular-weight hydrocarbons include n-hexane ($C_6H_{14}$) and n-decane ($C_{10}H_{22}$), and m > 10 may be satisfied.

The gas generated during heating of coal further contains a sulfur-based gas. The sulfur-based gas contains, for example, a gas component such as hydrogen sulfide ($H_2S$), carbonyl sulfide (COS), sulfur dioxide ($SO_2$), carbon disulfide ($CS_2$), or thiophene ($C_4H_4S$).

<<Preliminary heating step>>

Of the gas generated during heating of coal, gas generated at a specific heating temperature A or a heating temperature range B may be quantified.

In this case, it is preferable to previously provide a step (preliminary heating step) of heating coal at a temperature (preliminary heating temperature) lower than the heating temperature A or a lower limit temperature of the heating temperature range B and discharging the generated gas.

The preliminary heating temperature may be set in consideration of a control range of the temperature inside the pyrolysis furnace 8. For example, when the control range is smaller than 5°C, the preliminary heating temperature is set to be lower than the heating temperature A or the lower limit of the heating temperature range B by 5°C.

The time for heating coal at the preliminary heating temperature may be any period of time as long as the time is long enough for gas to be no longer generated from the coal by heating at the preliminary heating temperature, and is, for example, 10 minutes or more and preferably 15 minutes or more.

A discharge path of the gas generated in the preliminary heating step is not particularly limited. The gas may be discharged from a split path (not illustrated), or may be allowed to pass through the separation column 3 of the gas chromatograph and discharged.

When the gas is allowed to pass through the separation column 3, it is preferable to confirm that no gas is detected by the mass spectrometry unit 7 before the gas collection step.

In the case of quantifying the gas generated at the heating temperature A, after the preliminary heating step, the coal is heated in the pyrolysis furnace 8 set at the heating temperature A for a certain period of time to generate gas.

When the heating time is too short, the entire amount of coal is not sufficiently heated, and the entire amount of gas generated at the heating temperature A cannot be collected in some cases. Therefore, the heating time is preferably 10 minutes or more and more preferably 15 minutes or more.

In the case of quantifying the gas in the heating temperature range B, after the preliminary heating step, the temperature inside the pyrolysis furnace 8 is raised from the lower limit temperature to the upper limit temperature of the heating temperature range B to generate gas from the coal.

When the temperature rise rate is too high, the heating of the coal at each temperature in the temperature raising process is insufficient, and the entire amount of gas generated over the entire heating temperature range B cannot be collected in some cases. Therefore, the temperature rise rate is preferably 10°C/min or less and more preferably 5°C/min or less.

After the temperature raising process, a step of heating at an upper limit temperature may be added.

<Gas introduction step>

In the gas introduction step, the gas collected in the gas collection step is introduced into the separation column 3 of the gas chromatograph from the gas injection unit 6 by using a carrier gas.

The gas introduced into the separation column 3 flows together with the carrier gas from one end (the end on the gas injection unit 6 side) toward the other end (the end on the mass spectrometry unit 7 side) of the separation column 3.

In order to simulate a coke oven, the carrier gas is preferably an inert gas and more preferably a He gas having less influence in the mass spectrometry unit 7. The flow rate and the split ratio of the carrier gas are not particularly limited, and may be appropriately adjusted according to the analysis sensitivity of the mass spectrometry unit 7 or the like.

<Gas capturing step>

In the gas capturing step, the cooling unit 5 (for example, a cryotrap) is operated to cool at least a part, in the length direction, of the separation column 3 to a certain cooling temperature.

Hereinafter, for convenience, the portion of the separation column 3 cooled by the cooling unit 5 may be referred to as a "cooling target portion", and the portion of the separation column 3 that is not the cooling target portion may be referred to as a "non-cooling target portion".

The gas introduced into the separation column 3 is solidified in the cooling target portion of the separation column 3 by the operation of the cooling unit 5, and is captured inside the separation column 3.

For example, a case where gas to be introduced into the separation column 3 is a sulfur-based gas is considered. Among the gas components contained in the sulfur-based gas, since hydrogen sulfide has a lowest boiling point of -60°C, the cooling temperature of the separation column 3 is set to a temperature lower than - 60°C. As a result, while the cooling unit 5 is operating, all the gas components having boiling points of -60°C or higher are captured in the separation column 3.

A case where the gas introduced into the separation column 3 is a gas generated during heating of coal is considered.

Examples of gas components other than the sulfur-based gas among the gas components contained in the gas generated during heating of coal include hydrogen (boiling point: -252.6°C); low-molecular-weight hydrocarbons such as methane (boiling point: -162°C), ethylene (boiling point: - 103.7°C), and ethane (boiling point: -89°C); and high-molecular-weight hydrocarbons such as n-hexane (boiling point: 69°C) and n-decane (boiling point: 174°C).

Among them, a gas component (hydrogen, a low-molecular-weight hydrocarbon, or the like) having a boiling point lower than the cooling temperature of the separation column 3 passes through the separation column 3 without being captured by the separation column 3.

On the other hand, the gas components (a high-molecular-weight hydrocarbon and the like) having a boiling point equal to or higher than the cooling temperature of the separation column 3 are all captured in the separation column 3 together with the sulfur-based gas.

The time for cooling the cooling target portion of the separation column 3 is not particularly limited, and is, for example, about 30 seconds to 1 minute.

The temperature inside the chamber 2 (that is, the temperature of the non-cooling target portion of the separation column 3) is preferably 100°C or lower, more preferably 80°C or lower, and still more preferably 50°C or lower. The lower limit is not particularly limited, and is, for example, room temperature (for example, 25°C).

The temperature control inside the chamber 2 is performed by operating the heating unit 4.

When the temperature inside the chamber 2 is in the above range, the temperature gradient between the cooling target portion and the non-cooling target portion of the separation column 3 is gentle during the operation of the cooling unit 5. As a result, the influence range of the cooling unit 5 (that is, the cooling target portion of the separation column 3) is widened, and the analysis error in the mass spectrometry unit 7 can be reduced in the analysis step described below.

Also in the next gas releasing step, the temperature inside the chamber 2 is preferably in the above range for the reason described below.

<Gas releasing step>

In the gas releasing step, the captured gas is released by deactivating the cooling of the separation column 3.

The cooling is deactivated by turning off the operation of the cooling unit 5 (for example, a cryotrap). As a result, the temperature of the cooling target portion of the separation column 3 rises, and the solidified gas component volatilizes. At this time, the gas components are volatilized in order from a gas component having a lower boiling point, move forward inside the separation column 3, and are introduced into the mass spectrometry unit 7 with a time difference. The influence of the separation capacity due to the boiling point difference is larger than that of the separation capacity due to the column polarity.

The gas components reach the mass spectrometry unit 7 in ascending order of boiling point. Therefore, into the mass spectrometry unit 7, a low-molecular-weight hydrocarbon (hydrocarbon-based gas) is first introduced, then a sulfur-based gas is volatilized and introduced, and a high-molecular-weight hydrocarbon (hydrocarbon-based gas) is finally introduced.

A low-molecular-weight hydrocarbon-based gas such as methane, ethane, or ethylene has a smaller mass-to-charge ratio (m/z) than the sulfur-based gas. Therefore, a peak superposed with the sulfur-based gas does not occur.

On the other hand, a high-molecular-weight hydrocarbon-based gas generates fragment ions having the same mass-to-charge ratio (m/z) as that of the sulfur-based gas when ionized by the mass spectrometry unit 7. Therefore, a peak superposed with the sulfur-based gas occurs, and the peak of the sulfur-based gas cannot be separated with high accuracy.

However, in the present embodiment, the high-molecular-weight hydrocarbon-based gas is introduced into the mass spectrometry unit 7 after the sulfur-based gas. Therefore, the sulfur-based gas can be analyzed by the mass spectrometry unit 7 while avoiding superposition of peaks caused by the high-molecular-weight hydrocarbon-based gas containing no sulfur. That is, the sulfur-based gas generated during heating of coal can be quantified with high accuracy.

Meanwhile, in the gas releasing step, when the temperature inside the chamber 2 (the temperature of the non-cooling target portion of the separation column 3) is too high (for example, 300°C), the rate of temperature rise of the cooling target portion of the separation column 3 may become too high after the deactivation of the cooling.

In this case, not only the sulfur-based gas but also the high-molecular-weight hydrocarbon-based gas that affects the analysis of the sulfur-based gas immediately reaches the temperature inside the chamber 2 and is likely to volatilize. Then, in the mass spectrometry unit 7, the superposition of both the gases is likely to occur, and there is a possibility that the quantitative accuracy of the sulfur-based gas is insufficient.

Therefore, also in the gas releasing step, the temperature inside the chamber 2 is preferably in the above range. That is, the temperature inside the chamber 2 is preferably 100°C or lower, more preferably 80°C or lower, and still more preferably 50°C or lower.

With this, the rate at which the cooling target portion of the separation column 3 whose cooling has been deactivated rises in temperature can be made slow, and as a result, the sulfur-based gas having a lower boiling point volatilizes before the high-molecular-weight hydrocarbon-based gas that affects the analysis of the sulfur-based gas, and is easily introduced into the mass spectrometry unit 7. That is, it is easy to quantify the sulfur-based gas generated during heating of coal with high accuracy.

<Temperature rise heating step>

The present quantification method preferably further includes a temperature rise heating step of raising a temperature to heat the separation column 3 in addition to the gas releasing step.

Specifically, for example, the cooling of the separation column 3 is deactivated, and the temperature is raised to heat the separation column 3. The temperature rise heating of the separation column 3 is performed by operating the heating unit 4 to raise the temperature and heat the inside of the chamber 2.

The temperature rise rate at this time is, for example, 5°C/min or more.

On the other hand, for the reasons described above, the temperature rise rate is preferably not too high, and specifically, the temperature rise rate is preferably 20°C/min or less, more preferably 15°C/min or less, and still more preferably 10°C/min or less.

<Analysis step>

In the analysis step, the gas (sulfur-based gas) released in the gas releasing step is analyzed and quantified by the mass spectrometry unit 7.

The mass spectrometry unit 7 separates and specifies gas components contained in the sulfur-based gas released in the gas releasing step according to the mass-to-charge ratio.

In the present embodiment, with respect to hydrogen sulfide ($H_2S$, m/z: 34), carbonyl sulfide (COS, m/z: 60), sulfur dioxide ($SO_2$, m/z: 64), carbon disulfide ($CS_2$, m/z: 76), and thiophene ($C_4H_4S$, m/z: 84), which are main low-molecular-weight gas components contained in the sulfur-based gas, a mass spectrum with a single peak is obtained at each mass-to-charge ratio (m/z). The tendency of the amount of each gas component can be grasped by the magnitude of the peak area.

Separately, each gas component is quantified by using a peak area of each gas component having a known concentration obtained under the same conditions as a calibration curve.

After the analysis in the mass spectrometry unit 7 is completed, the heating unit 4 is preferably operated to heat the inside of the chamber 2 for the purpose of removing hydrocarbon remaining in the separation column 3. At this time, the temperature inside the chamber 2 is preferably 200°C or higher and more preferably 300°C or higher.

[Method for estimating amount of gas generated during coal carbonization and method for manufacturing coke]

Next, a method for estimating an amount of gas generated during coal carbonization according to the present embodiment (hereinafter, also referred to as "present estimation method") will be described.

The following description also serves as a description of a method for manufacturing coke according to the present embodiment (hereinafter, also referred to as "present manufacturing method").

In the present estimation method, first, the sulfur-based gas generated during heating of coal is quantified by performing the above-described present quantification method.

In this process, as shown in EXAMPLES described below, it is preferable to quantify each gas component (such as hydrogen sulfide or sulfur dioxide) contained in the sulfur-based gas generated during heating of coal.

It is also preferable to repeatedly perform the above-described present quantification method. In this case, as shown in EXAMPLES described below, in the gas collection step, coal is heated at different heating temperatures or in heating temperature ranges, and gas thereby generated is collected.

Based on the obtained quantitative results, the generation temperature or generation temperature range and the generation amount of sulfur-based gas generated during coal carbonization in a coke oven are estimated.

When n kinds of coal are blended, charged into a coke oven, and subjected to carbonization, a generation amount F(T) of sulfur-based gas is represented by, for example, the following formula.

$$F(T) = A_1 \times f_1(T) + A_2 \times f_2(T) + \cdots + A_n \times f_n(T)$$

In the above formula, $f_n(T)$ is a function of temperature indicating the amount of sulfur-based gas generated from coal, and $A_n$ is a blending ratio of coal.

The operation of the coke oven is performed by operating a plurality of coke ovens.

Therefore, focusing on $F(T)$ of each coke oven, the operating times of the respective coke ovens are adjusted so that the amounts of sulfur-based gas generated are distributed with respect to time.

With this configuration, it is possible to prevent a high-concentration sulfur-based gas from locally flowing into a desulfurization facility provided downstream of each coke oven, and the operation of the coke oven can be stabilized.

In addition, when coke is actually manufactured by coal carbonization, a manufacturing condition of coke such as a coal blending ratio can be modified based on an estimation result obtained by the present estimation method. The estimation result can also be utilized for selection of coal suitable for coke oven operation.

EXAMPLES

Hereinafter, the present invention will be specifically described with reference to Examples. However, the present invention is not limited to Examples described below.

According to the method described in JIS M 8811 "Coal and coke - Sampling and sample preparation", each of nine kinds of coal (Coals 1 to 9) was sampled in an amount of 100 g. Each coal was pulverized and classified to 125 um or less and air-dried at 40°C for 4 hours.

The total sulfur amounts (unit: mass%) of Coals 1 to 9 are shown in Table 1 below. The total sulfur amount is a value obtained by performing measurement using a high temperature combustion method described in JIS M 8813 "Coal and coke - Determination of constituents" and converting the measured value into a mass value per 1 g of coal.

[Table 1]

Table 1

| | Total sulfur amount [mass%] |
| --- | --- |
| Coal 1 | 0.48 |
| Coal 2 | 0.50 |
| Coal 3 | 0.43 |
| Coal 4 | 2.05 |
| Coal 5 | 0.82 |
| Coal 6 | 0.56 |
| Coal 7 | 0.38 |
| Coal 8 | 0.95 |
| Coal 9 | 0.40 |

For Coals 1 to 9 shown in Table 1 above, the sulfur-based gas generated during heating of coal was quantified using the apparatus 1 described based on FIG. 1.

More specifically, the generation amounts of respective gas components (hydrogen sulfide, carbonyl sulfide, sulfur dioxide, carbon disulfide, and thiophene) were determined for each heating temperature range in a range of 300 to 700°C at intervals of 50°C.

As the cooling unit 5, a cryotrap was used. As the gas injection unit 6 and the pyrolysis furnace 8, a pyrolyzer was used. Each coal was collected in an amount of 5 mg and charged into the pyrolysis furnace 8.

The atmosphere in the furnace of the pyrolysis furnace 8 was set to an atmosphere of He gas being a carrier gas. The He gas was introduced from the pyrolysis furnace 8 into the gas injection unit 6, the separation column 3, and the mass spectrometry unit 7 at a flow rate of 1 L/min and a split ratio of 1 : 50.

First, each gas component was quantified in a heating temperature range of 300 to 350°C.

First, the coal charged into the pyrolysis furnace 8 was heated at a preliminary heating temperature of 295°C for 10 minutes. As a result, the attached moisture and the gas generated in a heating temperature range of 295°C or lower were discharged through a split path (or a path passing through the separation column 3) (preliminary heating step).

Thereafter, the coal was moved to a retreat place of the pyrolysis furnace 8, and then the temperature inside the pyrolysis furnace 8 was raised to 300°C.

Next, while the heating unit 4 was operated to bring the temperature inside the chamber 2 (that is, the temperature of the non-cooling target portion of the separation column 3) to 40°C, the cooling unit 5 (cryotrap) was operated to cool the cooling target portion of the separation column 3 to -180°C.

In this state, the coal was returned to the furnace of the pyrolysis furnace 8, the temperature in the furnace was raised at 3°C/min to reach 350°C, and then the coal was held for 5 minutes. In this way, gas was generated from the coal (gas collection step). Thereafter, the coal was moved to the retreat place of the pyrolysis furnace 8.

The gas generated from the coal was introduced into the separation column 3 from the gas injection unit 6 using a carrier gas (gas introduction step), and solidified in the cooling target portion of the separation column 3 cooled to -180°C (gas capturing step). The cooling of the separation column 3 was continuously performed during the charging of the coal into the furnace of the pyrolysis furnace 8.

Subsequently, the operation of the cooling unit 5 was turned off to release the cooling of the separation column 3 (gas releasing step). At the same time, the heating unit 4 was operated to increase the temperature inside the chamber 2 to 300°C at 10°C/min (temperature rise heating step).

The gas solidified in the cooling target portion of the separation column 3 volatilized, moved by the carrier gas in the ascending order of boiling point, and reached the mass spectrometry unit 7. The gas having reached the mass spectrometry unit 7 was separated according to the mass-to-charge ratio, and components thereof were specified (analysis step).

With respect to hydrogen sulfide ($H_2S$, m/z: 34), carbonyl sulfide (COS, m/z: 60), sulfur dioxide ($SO_2$, m/z: 64), carbon disulfide ($CS_2$, m/z: 76), and thiophene ($C_4H_4S$, m/z: 84) generated during heating of coal, a mass spectrum with a single peak was obtained at each mass-to-charge ratio.

The peak area of each gas component was converted using a calibration curve prepared in advance (the peak area of each gas component having a known concentration obtained under the same conditions), thus quantifying each gas component.

Next, the heating unit 4 was operated to heat the inside of the chamber 2 at 300°C for 30 minutes. As a result, hydrocarbon remaining inside the separation column 3 was removed. Thereafter, the inside of the chamber 2 was cooled to 40°C.

Subsequently, each gas component was quantified in a heating temperature range of 350 to 400°C.

First, in a state where the temperature inside the chamber 2 was 40°C, the cooling unit 5 was operated to cool the cooling target portion of the separation column 3 to -180°C. The coal having been moved to the retreat place of the pyrolysis furnace 8 was introduced into the pyrolysis furnace 8 set at 350°C, which was the upper limit of the previous heating temperature range (300 to 350°C), and heated for 5 minutes (preliminary heating step).

Next, the temperature inside the pyrolysis furnace 8 was raised at 3°C/min to reach 400°C, and then the coal was held for 5 minutes. In this way, gas was generated from the coal (gas collection step). Thereafter, the coal was moved to the retreat place of the pyrolysis furnace 8.

In the subsequent steps, each gas component was quantified in the same manner as in the step in the previous heating temperature range of 350 to 400°C.

In this way, each gas component was quantified in a range of 300 to 700°C while the heating temperature range was shifted at intervals of 50°C.

Based on the obtained quantitative results, the generation amounts of the respective gas components generated during heating of Coals 1 to 9 were organized for each heating temperature range.

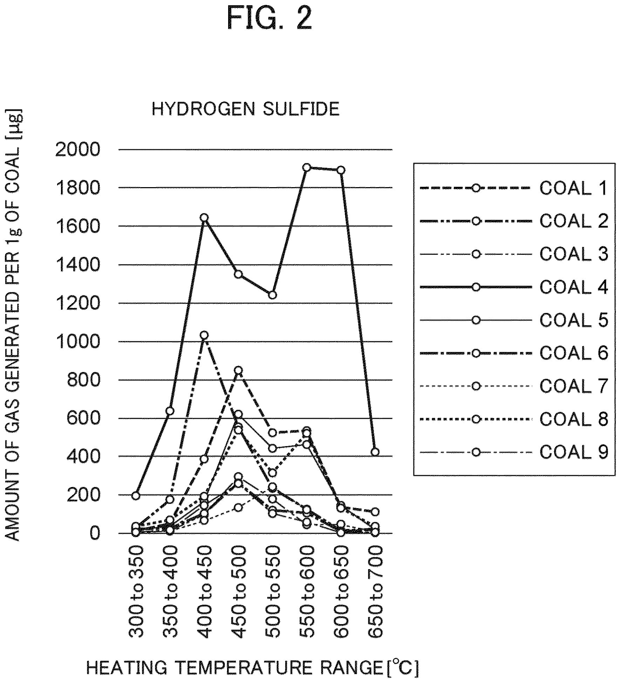

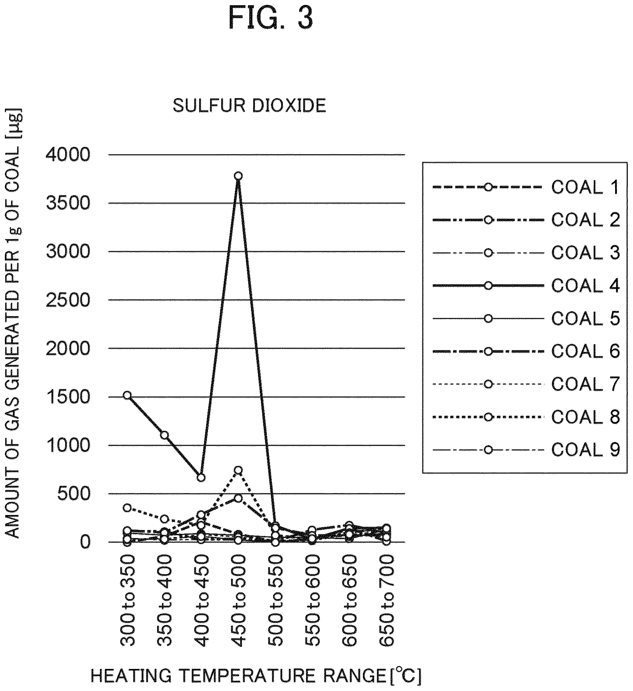

As representatives, the results of hydrogen sulfide ($H_2S$) are shown in Table 2 below and the graph of FIG. 2, and the results of sulfur dioxide ($SO_2$) are shown in Table 3 and the graph of FIG. 3.

[Table 2]

Table 2

| Heating temperature range [°C] | 300 to 350 | 350 to 400 | 400 to 450 | 450 to 500 | 500 to 550 | 550 to 600 | 600 to 650 | 650 to 700 |
|---|---|---|---|---|---|---|---|---|
| Coal 1 | 12.67 | 50.17 | 387.39 | 848.94 | 522.24 | 535.85 | 137.34 | 112.85 |
| Coal 2 | 28.04 | 174.39 | 1033.11 | 554.81 | 233.06 | 125.74 | 14.23 | 23.96 |
| Coal 3 | 7.66 | 12.58 | 98.71 | 293.93 | 178.30 | 45.70 | 46.49 | 7.78 |
| Coal 4 | 195.77 | 636.12 | 1643.50 | 1350.47 | 1240.47 | 1905.45 | 1892.52 | 424.13 |
| Coal 5 | 18.73 | 37.82 | 165.72 | 619.49 | 443.17 | 461.72 | 145.69 | 21.43 |
| Coal 6 | 21.92 | 26.49 | 102.51 | 262.93 | 120.70 | 106.29 | 10.74 | 16.51 |
| Coal 7 | 2.99 | 11.07 | 65.82 | 133.88 | 241.81 | 122.85 | 3.78 | 3.91 |
| Coal 8 | 36.69 | 69.99 | 191.75 | 538.72 | 314.23 | 521.35 | 131.07 | 37.72 |
| Coal 9 | 5.64 | 16.85 | 145.17 | 258.72 | 103.42 | 58.42 | 3.50 | 7.07 |

Hydrogen sulfide ($H_2S$)

Generation amount per 1 g of coal [μg/g]

[Table 3]

Table 3

| | Heating temperature range [°C] | 300 to 350 | 350 to 400 | 400 to 450 | 450 to 500 | 500 to 550 | 550 to 600 | 600 to 650 | 650 to 700 |
|---|---|---|---|---|---|---|---|---|---|
| Sulfur dioxide ($SO_2$) Generation amount per 1 g of coal [μg/g] | Coal 1 | 0.00 | 59.96 | 208.74 | 84.69 | 6.08 | 28.23 | 141.61 | 13.03 |
| | Coal 2 | 114.43 | 112.36 | 25.77 | 32.22 | 7.90 | 125.51 | 176.50 | 95.99 |
| | Coal 3 | 36.26 | 20.14 | 30.16 | 21.28 | 0.00 | 31.87 | 121.75 | 111.77 |
| | Coal 4 | 1517.73 | 1106.61 | 671.40 | 3781.99 | 172.27 | 30.35 | 141.28 | 148.35 |
| | Coal 5 | 94.08 | 69.63 | 86.10 | 69.62 | 51.21 | 43.71 | 36.47 | 126.52 |
| | Coal 6 | 123.70 | 101.12 | 283.72 | 456.71 | 147.58 | 73.41 | 63.12 | 80.87 |
| | Coal 7 | 29.89 | 35.05 | 77.01 | 54.84 | 0.65 | 16.91 | 78.84 | 113.89 |
| | Coal 8 | 358.42 | 239.09 | 176.53 | 743.72 | 73.89 | 28.87 | 100.89 | 142.77 |
| | Coal 9 | 33.05 | 30.98 | 60.28 | 23.67 | 0.00 | 36.71 | 83.18 | 53.12 |

The outline of the graph varies depending on the brand of coal, but a large tendency is as follows.

As for hydrogen sulfide ($H_2S$), most of the coal had a peak of the amount of hydrogen sulfide generated at each of a low temperature side and a high temperature side with the 500°C boundary (see Table 2 and FIG. 2).

As for sulfur dioxide ($SO_2$), two peaks were generated on a low temperature side and a high temperature side with the boundary around 550°C, and Coal 4, Coal 6, and Coal 8 showed particularly strong peaks at around 450°C (see Table 3 and FIG. 3).

As described above, it was found that the generation amount of sulfur-based gas generated during heating of coal greatly changes depending on the heating temperature.

This is considered to be caused by a difference in the existence form of sulfur in coal.

Sulfur-based gases generated during heating of coal have various generation temperatures depending on the existence form of sulfur in the coal. For example, the peak of sulfur dioxide observed at around 500°C is considered to have appeared due to decomposition of sulfate sulfur. From these results, the amount of sulfur-based gas generated when coke is actually manufactured by coal carbonization is presumed to vary depending on the temperature during coal carbonization.

As described above, for each brand of coal, the generation amounts of sulfur-based gas can be organized for each generation temperature range. Therefore, for example, the coal blending ratio can be set in advance so that a large amount of sulfur-based gas is not generated at a certain temperature, and contribution to stable manufacturing of coke is expected.

DESCRIPTION OF SYMBOLS

1: apparatus used for quantification of gas generated from coal
2: chamber
3: separation column
4: heating unit
5: cooling unit
6: gas injection unit
7: mass spectrometry unit
8: pyrolysis furnace

**Claims**

1. A method for quantifying gas generated from coal, comprising:

   a gas collection step of collecting gas generated during heating of coal;
   a gas introduction step of introducing the gas collected in the gas collection step into a separation column of a gas chromatograph;
   a gas capturing step of capturing the gas introduced into the separation column by cooling the separation column;
   a gas releasing step of releasing the gas captured in the gas capturing step by deactivating the cooling of the separation column; and
   an analysis step of analyzing and quantifying the gas released in the gas releasing step by a mass spectrometry unit.

2. The method for quantifying gas generated from coal according to claim 1, further comprising a temperature rise heating step of raising a temperature to heat the separation column the cooling of which has been deactivated.

3. The method for quantifying gas generated from coal according to claim 1 or 2,
   wherein the gas contains a sulfur-based gas.

4. A method for estimating an amount of gas generated during coal carbonization, comprising estimating a generation temperature or generation temperature range and a generation amount of a sulfur-based gas generated during coal carbonization based on a quantitative result obtained by the method for quantifying according to claim 3.

5. A method for manufacturing coke by subjecting coal to carbonization,
   wherein a manufacturing condition of coke is modified based on an estimation result obtained by the method for estimating according to claim 4.

6. The method for manufacturing coke according to claim 5, wherein the manufacturing condition is a coal blending ratio.

# FIG. 1

# FIG. 2

HYDROGEN SULFIDE

Legend:
- COAL 1
- COAL 2
- COAL 3
- COAL 4
- COAL 5
- COAL 6
- COAL 7
- COAL 8
- COAL 9

Y-axis: AMOUNT OF GAS GENERATED PER 1g OF COAL [μg] (0, 200, 400, 600, 800, 1000, 1200, 1400, 1600, 1800, 2000)

X-axis: HEATING TEMPERATURE RANGE[°C] (300 to 350, 350 to 400, 400 to 450, 450 to 500, 500 to 550, 550 to 600, 600 to 650, 650 to 700)

# FIG. 3

SULFUR DIOXIDE

## INTERNATIONAL SEARCH REPORT

| International application No. |
|---|
| **PCT/JP2022/042941** |

**A. CLASSIFICATION OF SUBJECT MATTER**

*G01N 30/08*(2006.01)i; *C10B 57/04*(2006.01)i; *G01N 27/62*(2021.01)i; *G01N 30/06*(2006.01)i; *G01N 30/72*(2006.01)i; *G01N 33/22*(2006.01)i

FI: G01N30/08 G; C10B57/04; G01N30/72 A; G01N30/06 G; G01N33/22 A; G01N27/62 V; G01N27/62 C

According to International Patent Classification (IPC) or to both national classification and IPC

**B. FIELDS SEARCHED**

Minimum documentation searched (classification system followed by classification symbols)

G01N30/08; C10B57/04; G01N27/62; G01N30/06; G01N30/72; G01N33/22

Documentation searched other than minimum documentation to the extent that such documents are included in the fields searched

Published examined utility model applications of Japan 1922-1996
Published unexamined utility model applications of Japan 1971-2023
Registered utility model specifications of Japan 1996-2023
Published registered utility model applications of Japan 1994-2023

Electronic data base consulted during the international search (name of data base and, where practicable, search terms used)

JSTPlus/JMEDPlus/JST7580 (JDreamIII)

**C. DOCUMENTS CONSIDERED TO BE RELEVANT**

| Category* | Citation of document, with indication, where appropriate, of the relevant passages | Relevant to claim No. |
|---|---|---|
| Y | JP 2009-257920 A (NIPPON STEEL CORP.) 05 November 2009 (2009-11-05)<br>claim 1, paragraphs [0033]-[0049] | 1-6 |
| Y | SCDによるガス中硫黄化合物分析, 第342回ガスクロマトグラフィー研究懇親会, 04 March 2016, pp. 1-4, non-official translation (Analysis of sulfur compounds in gas by SCD. The 342nd Gas Chromatography Conference.)<br>p. 4, 「コールドトラップ温度の影響」, non-official translation ("effect of cold trap temperature") | 1-6 |
| Y | JP 2014-529080 A (WUHAN TIANHONG INSTRUMENTS CO., LTD.) 30 October 2014 (2014-10-30)<br>claims 1-7 | 1-6 |

☐ Further documents are listed in the continuation of Box C.　　☑ See patent family annex.

| * Special categories of cited documents: | "T" later document published after the international filing date or priority date and not in conflict with the application but cited to understand the principle or theory underlying the invention |
|---|---|
| "A" document defining the general state of the art which is not considered to be of particular relevance | |
| "E" earlier application or patent but published on or after the international filing date | "X" document of particular relevance; the claimed invention cannot be considered novel or cannot be considered to involve an inventive step when the document is taken alone |
| "L" document which may throw doubts on priority claim(s) or which is cited to establish the publication date of another citation or other special reason (as specified) | "Y" document of particular relevance; the claimed invention cannot be considered to involve an inventive step when the document is combined with one or more other such documents, such combination being obvious to a person skilled in the art |
| "O" document referring to an oral disclosure, use, exhibition or other means | |
| "P" document published prior to the international filing date but later than the priority date claimed | "&" document member of the same patent family |

| Date of the actual completion of the international search | Date of mailing of the international search report |
|---|---|
| **31 January 2023** | **07 February 2023** |

| Name and mailing address of the ISA/JP | Authorized officer |
|---|---|
| **Japan Patent Office (ISA/JP)**<br>**3-4-3 Kasumigaseki, Chiyoda-ku, Tokyo 100-8915**<br>**Japan** | |
| | Telephone No. |

Form PCT/ISA/210 (second sheet) (January 2015)

## INTERNATIONAL SEARCH REPORT
### Information on patent family members

International application No.

**PCT/JP2022/042941**

| Patent document cited in search report | | | Publication date (day/month/year) | Patent family member(s) | | | Publication date (day/month/year) |
|---|---|---|---|---|---|---|---|
| JP | 2009-257920 | A | 05 November 2009 | (Family: none) | | | |
| JP | 2014-529080 | A | 30 October 2014 | US | 2014/0318217 | A1 | |
| | | | | claims 1-7 | | | |
| | | | | EP | 2757369 | A1 | |
| | | | | CN | 102375041 | A | |

Form PCT/ISA/210 (patent family annex) (January 2015)

**REFERENCES CITED IN THE DESCRIPTION**

**Patent documents cited in the description**

- JP 2009257920 A